(19) Europäisches Patentamt

European Patent Office

Office européen des brevets

(11) Publication number: **0 302 536 B1**

(12) **EUROPEAN PATENT SPECIFICATION**

(45) Date of publication of patent specification: **19.11.92**   (51) Int. Cl.⁵: **A61L  15/00**

(21) Application number: **88201241.2**

(22) Date of filing: **22.04.83**

(60) Publication number of the earlier application in accordance with Art.76 EPC: **0 092 999**

(54) **Granules for use in treating wounds.**

(30) Priority: **22.04.82 US 370893**

(43) Date of publication of application:
**08.02.89 Bulletin  89/06**

(45) Publication of the grant of the patent:
**19.11.92 Bulletin  92/47**

(84) Designated Contracting States:
**AT BE CH DE FR GB IT LI LU NL SE**

(56) References cited:
**EP-A- 0 047 647     EP-A- 0 048 323
EP-A- 0 075 791     FR-A- 2 215 230
FR-A- 2 302 752     GB-A- 2 061 732
US-A- 3 972 328     US-A- 4 192 785
US-A- 4 225 580     US-A- 4 292 972**

**CHEMICAL ABSTRACTS, vol. 98, no. 9, February 1983, page 28, abstract no. 65221r, Columbus, Ohio, US; G. FURNADZHIEV et al.: "Experimental study of granulated pectin with a moderate degree of esterification for antiinflammatory activity", & PROBL. VUTR. MED. 1981, 9(2), 67-72**

(73) Proprietor: **E.R. SOUIBB & SONS, INC.
Lawrenceville-Princeton Road
Princeton New Jersey 08543-400(US)**

(72) Inventor: **Pawelchak, John M.
J-20 Avon Drive
East Windsor(US)**
Inventor: **Freeman, Frank M.
10 Brandon Road
Lawrenceville New Jersey(US)**

(74) Representative: **Thomas, Roger Tamlyn et al
D. Young & Co. 10 Staple Inn
London WC1V 7RD(GB)**

**Description**

This invention relates to particulate products for the treatment of wound exudate.

EP-A-48323 describes a powder for treating wound extrudate which is a cross-linked hydrophilic acrylic or methacrylic acid derivative polymer permeated by a gellable polysaccharide and/or protein polypeptide.

US-A-4225580 describes dry particles of a water-insoluble hydrophilic polymer capable of limited swelling to form a gel with low molecular weight wound exudate components but incapable of absorbing high molecular weight components such as fibrinogen. The preferred polymer is dextran (or a dextran derivative) cross-linked with epichlorohydrin. The product is removed from the wound while still in particulate form.

US-A-4292972 describes a lyophilized foam sponge product having hemostatic properties and formed from the hydrocolloids, gelatin, pectin and carboxymethylcellulose. There is a reference at one point in the specification to the possibility of converting the lyophilized sponge to a granular product by passing it through a screen.

US-A-3972328 describes a bandage having an adhesive layer in which hydrocolloid is dispersed.

Chem. Abst., Vol. 98, 65221r discloses the external application of granulated pectin as a 4% jelly to stimulate the epithelialization of wounds.

According to the present invention there is provided a granular product for treating wound exudate, having a particle size of from about 10 to about 40 mesh (from 2.00 to 0.42 mm sieve openings) consisting essentially of one or more water dispersible hydrocolloids selected from pectin, gelatin, sodium carboxymethylcellulose, collagen, guar gum, locust bean gum, karaya, and calcium carboxymethylcellulose, wherein the granular product is prepared from the above powder ingredients by a dry compaction, wet granulation, or fluidized bed granulation technique.

The granular product is preferably used with an occlusive wound dressing. Embodiments of the wound dressing and the granular product will now be described with reference to the accompanying drawings.

Fig. 1 is an overall view of one embodiment of the composite dressing (greatly enlarged).

Fig. 2 is a front view along line 2-2 of Fig. 1.

Fig. 3 is an overall view of another embodiment of the composite dressing (greatly enlarged).

Fig. 4 is a front view along line 3-3 of Fig. 3.

Fig. 5 is an overall view of another embodiment of the composite dressing (greatly enlarged).

Fig. 6 is a front view along line 5-5 of Fig. 5.

Fig. 7 is a top view of an open wound covered by the dressing and partially cut-away to show the granular packing.

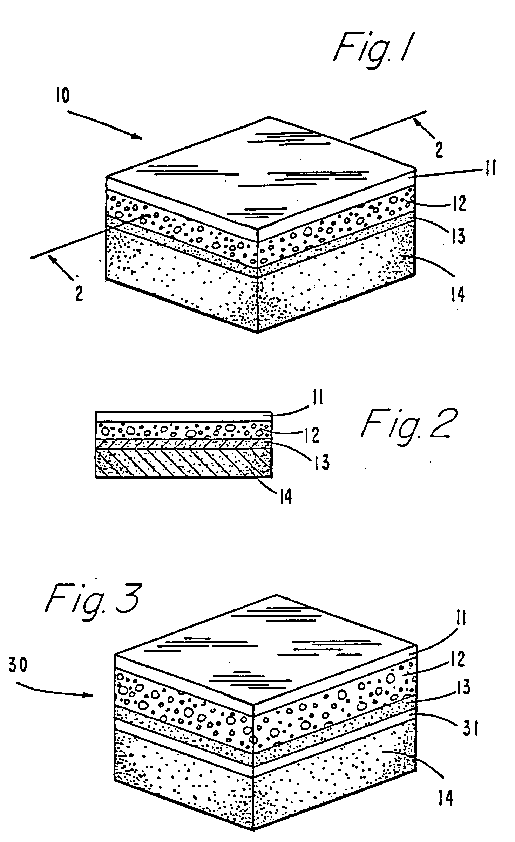

One embodiment of the occlusive dressing 10 as shown in figs 1 and 2 comprises a first adhesive layer 14 which is formulated from materials selected to interact with fluid discharged from the wound as well as forming a fluid-tight bond with the healthy skin around the wound so as to seal the dressing to the skin. The dressing of this embodiment includes a second adhesive layer 13 formulated of materials which can be cast onto foam layer 12 and will form an aggressive bond when pressed into contact with adhesive layer 14. The layer 12 is a semi-open cell elastic or flexible foam. The outer layer 11 can be a polymeric film or a skin formed on the top of foam layer 12 which serves to protect the exposed surface of the dressing from contamination by water or soil.

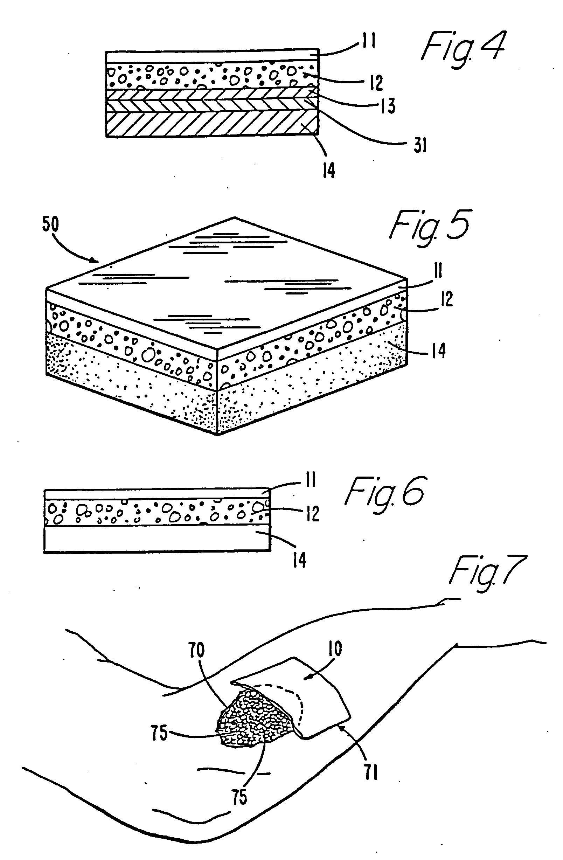

An alternative embodiment of the occlusive dressing is shown as 30 in figs. 3 and 4. Dressing 30 includes a layer of deodorizing material designated 31 interposed between adhesive layers 14 and 13.

Another alternative embodiment of the occlusive dressing is shown as 50 in figs. 5 and 6. Dressing 50 omits second adhesive layer 13 and has adhesive layer 14 laminated directly to the semi-open cell elastic foam 12.

Adhesive layer 14 comprises a homogeneous blend of one or more pressure sensitive adhesive materials and one or more water dispersable hydrocolloidal materials. Adhesive layer 13 comprises a homogeneous blend of one or more pressure sensitive adhesive materials, one or more water dispersable hydrocolloidal materials, a tackifier, and a plasticizer or solvent. Additionally, one or more thermoplastic elastomers may be included with the pressure sensitive adhesive materials in either or both of layers 14 and 13 and one or more water swellable cohesive strengthening agents and/or one or more natural or synthetic polymers capable of developing elastomeric properties when hydrated may be included with the hydrocolloidal materials in either or both of layers 14 and 13.

Suitable pressure sensitive adhesive materials for inclusion in layers 14 and 13 are various natural or synthetic viscous or elastomeric substances such as natural rubber, silicone rubber, acrylonitrile rubber, polyurethane rubber, polyisobutylene, etc. Low molecular weight polyisobutylenes having a viscosity average molecular weight of from about 36,000 to about 58,000 (Florey) possessing pressure sensitive adhesive properties are preferred. Such polyisobutylenes are commercially available under the trademark Vistanex from Exxon as grades LM-MS and LM-MH.

Optionally, one or more thermoplastic elastomers can be included in the pressure sensitive adhesive component of layers 14 and 13. These elastomers impart the properties of rubber-like extensibility and both rapid and complete recovery from modular strains to the pressure sensitive adhesive component. Suitable thermoplastic elastomers include medium molecular weight polyisobutylenes having a viscosity average molecular weight of from about 1,150,000 to 1,600,000 (Florey), butyl rubber which is a copolymer of isobutylene with a minor amount of isoprene having a viscosity average molecular weight of from about 300,000 to about 450,000 (Florey), and styrene copolymers such as styrene-butadiene-styrene (S-B-S), styrene-isoprene-styrene (S-I-S), and styrene-ethylene/butylene-styrene (S-EB-S) which are commercially available, for example, from Shell Chemical Co. under the trademark Kraton as Kraton D1100, D1102, Kraton D1107, Kraton 4000, Kraton G1600, and Kraton G4600. Preferred thermoplastic elastomers are butyl rubber having a viscosity average molecular weight of about 425,000 (commercially available from Exxon as grade 077), polyisobutylene having a viscosity average molecular weight of about 1,200,000 (commercially available under the trademark Vistanex from Exxon as grade L-100), and styrene-isoprene-styrene (S-I-S)-copolymers (commercially available from Shell as Kraton D1107).

The pressure sensitive adhesive component including the optional thermoplastic elastomer is present in adhesive layers 14 and 13 at from about 30% to about 70% by weight of the composition, preferably from about 35% to about 50% by weight. The thermoplastic elastomer can be employed at up to three times the weight of the pressure sensitive elastomeric substances but preferably the theromoplastic elastomer if present will be at from about 20% to about 150% by weight of the pressure sensitive elastomeric substance.

Adhesive layers 14 and 13 include from about 10% to about 65% by weight of one or more water dispersable hydrocolloid materials. Suitable hydrocolloid materials include sodium or calcium carboxymethylcellulose, pectin, gelatin, guar gum, locust bean gum, collagen and gum karaya. Adhesive layers 14 and 13 may also include up to about 50% by weight of one or more water swellable cohesive strengthening agents and/or one or more natural or synthetic polymers capable of developing elastomeric properties when hydrated provided that the water soluble hydrocolloid gums, water swellable cohesive strengthening agents, and hydratable polymers together are present at no more than about 70% by weight of adhesive layers 14 and 13. Suitable water swellable cohesive strengthening agents include finely divided substantially water insoluble cross-linked sodium carboxy-methylcellulose such as that commercially available under the trademark Aqualon or that described in U.S. Patent 3,589,364 and available commercially from the Buckeye Cellulose Corp., finely divided substantially water insoluble starch-acrylonitrile graft copolymer such as that described in U.S. Patent 3,661,815 and commercially available from the Grain Processing Corp., and finely divided substantially water insoluble cross-linked dextran such as that commercially available under the trademark Sephadex. Suitably hydratable polymers are gluten and long chain polymers of methyl vinyl ether/maleic acid, preferably, the long chain polymers of methyl vinyl ether/maleic acid commercially available under the trademark Gantrez from GAF Inc. The maleic acid moiety in the polymer may be intact (Gantrez S-97), may be an anhydride (Gantrez AN-169), or may be a metal salt such as the mixed sodium/calcium salts (Gantrez AT-955).

Preferably, the water dispersable hydrocolloids, the optional water swellable cohesive strengthening agents, and the optional hydratable polymers are present at from about 45% to about 65% by weight of adhesive layer 14 and from about 30% to about 50% by weight of adhesive layer 13.

The water dispersable hydrocolloids provide wet tack for adhesive layers 14 and 13 while the pressure sensitive adhesive component provides dry adhesion and imparts structural integrity to layers 14 and 13.

Adhesive layer 13 also includes from about 5% to about 15% by weight of a plasticizer or solvent such as mineral oil or petrolatum with mineral oil being preferred and from about 15% to about 25% by weight of a tackifier such as a terpene resin.

Small amounts, i.e., less than 5% by weight, of other ingredients may be included within adhesive layers 14 and 13. For example, an antioxidant such as butylated hydroxyanisole or butylated hydroxytoluene, a deodorant such as chlorophyllins, or a perfume agent may be included. In addition, small amounts of a pharmacologically active ingredient can be included in adhesive layer 14. For example, an antibiotic or antimicrobial agent such as neomycin, an antiseptic agent such as povidone iodine, and an antiinflammatory agent such as hydrocortisone or triamcinolone acetonide.

The semi-open cell elastic or flexible foam layer 12 can be formed from various elastomer materials such as polyester or polyether polyurethane foams, styrene-butadiene foams, certain rubber based foam, etc. The material should, of course, be non-toxic and stable. The preferred material is a flexible polyurethane foam having from about 50 to about 100 cells per linear inch with about 80 cells per linear inch being most preferred. By semi-open it is meant that the percentage of open or ruptured cells is from about 30 to about 85%.

The outer layer 11 can be a polymeric elastic or flexible film coating formed from a water impermeable pliable elastomer material such as flexible polyurethane, polyacrylate, polyethylene,etc. Layer 11 can be a skin of such polymeric material flame laminated to the top of foam layer 12 by means of heat and/or pressure. The exposed sides of polymeric foam layer 12 can also be coated or heat and/or pressure treated to form an impermeable film or skin. Polyurethane is the preferred material for film or skin 11.

In a typical dressing, adhesive layer 14 will vary in thickness from about 0.02 inches (0.5 mm) to about 0.1 inches (2.54 mm), preferably about 0.05 inches (1.27 mm), adhesive layer 13 will vary in thickness from about 0.005 inches (0.127 mm) to about 0.02 inches (0.5 mm), preferably about 0.015 inches (0.38 mm), foam layer 12 will vary in thickness from about 0.03 inches (0.76 mm) to about 0.1 inches (2.54 mm), preferably about 0.065 inches (1.65 mm) and outer layer or skin 11 will vary in thickness from about 0.001 inches (0.0254 mm) to about 0.003 inches (0.076 mm), preferably about 0.002 inches (0.05 mm).

As shown in figs. 3 and 4, a layer of deodorizing material can be included between the two adhesive layers. Suitable deodorizing materials include a sheet of foamed polymeric material such as polyurethane having a large number of activated carbon particles bound to the matrix of the foam. Such a material is commercially available under the tradename Bondina. Another type of deodorizing material is a paper or felt like substance containing activated carbon such as that commercially available under the tradename K-felt (Toyobo) or Getter paper (Mead). The layer of deodorizing material will vary from about .010 to 0.100 inches (0.254 to 2.54 mm).

The dressing 10 is prepared as follows. First, adhesive layer 14 is prepared by forming a homogeneous dispersion of the pressure sensitive adhesive material and any thermoplastic elastomer with a heavy duty mixer, e.g., a kneader mixer or sigma blade mixer. The hydrocolloid gums, water swellable cohesive strengthening agents, hydratable polymers, and any other optional ingredients are added and mixing continued until a homogeneous dough is formed. Alternatively, if layer 14 includes a thermoplastic elastomer,then the elastomer can first be broken down by mixing for several minutes, a portion of the pressure sensitive adhesive material and other ingredients added and mixing continued until a homogeneous mass is formed. The balance of pressure sensitive adhesive is then added and the mixing continued until a homogeneous dough is formed. This dough is then extruded into a thick slab which is thinned down by pressure rollers to the desired thickness.

Next, adhesive layer 13 is prepared by forming a mixture of the hydrocolloid gums, pressure sensitive adhesive materials, tackifier and plasticizer, as well as other optional ingredients such as thermoplastic elastomers, water swellable cohesive strengthening agents, hydratable polymers, antioxidants, etc., in an organic solvent such as heptane or hexane. The resulting adhesive slurry is then applied to a web of silicone coated release paper and the solvent is evaporated. Upon drying the hydrocolloids are dispersed throughout the adhesive layer. This adhesive material is then compressed with a laminate of semi-open cell flexible polymeric foam having a water impermeable polymeric coating or skin on one side.

Finally, the silicone coated release paper is stripped away from adhesive layer 13 and adhesive layers 13 and 14 are pressed together with heat to form the dressing. Silicone coated release paper can then be applied to the exposed surface of adhesive layer 14 and the dressing can be cut to the desired shape and packaged. After packaging, the dressing can be sterilized by gamma irradiation.

Dressing 30 is prepared in a similar manner except that a layer of deodorizing material is laminated between adhesive layers 14 and 13. Alternatively, dressing 30 can be prepared by first laminating adhesive layer 13 directly to the layer of deodorizing material 31 and then attaching this adhesive coated material to foam layer 12. In the case of dressing 50, adhesive layer 14 is prepared as described above and is then laminated while warm directly to foam layer 12 by means of pressure.

The following are representative examples of dressings

Example 1

A dressing was prepared having the following composition

Layers 11 and 12

Semi-open cell polyurethane foam having a polyurethane skin flame laminated to one surface.

Layer 13

4

| | Percent by weight |
|---|---|
| Polyisobutylene (Vistanex LM-MH) | 18.0 |
| Polyisobutylene (Vistanex L-100) | 20.0 |
| Terpene resin (Piccolyte) | 20.0 |
| Butylated hydroxytoluene | 0.5 |
| Mineral oil | 8.5 |
| Sodium carboxymethylcellulose | 18.0 |
| Gelatin | 15.0 |

Layer 14

| | Percent by weight |
|---|---|
| Polyisobutylene (Vistanex LM-MH) | 40 |
| Gelatin | 20 |
| Pectin | 20 |
| Sodium carboxymethylcellulose | 20 |

Adhesive layer 14 was prepared as follows. A premix was prepared by blending 1.4 kg. of gelatin, 1.4 kg. of pectin, and 1.4 kg. of sodium carboxymethylcellulose. The blended premix was added to a heavy duty sigma blade-type mixer followed by the addition of 1.4 kg. of polyisobutylene. After mixing for 10 minutes, an additional 1.4 kg. of polyisobutylene was added and mixing continued until a homogeneous dough was formed (about 10 to 20 minutes). This dough mass while hot and soft was extruded and thinned down by pressure rollers to a thickness of about 0.05 inches (1.27 mm).

A laminate of adhesive layer 13 to semi-open cell polymeric foam (layers 12 and 11) was prepared as follows.

A mixture of 0.31 kg. of polyisobutylene (Vistanex L-100), 0.28 kg. of sodium carboxymethylcellulose, and 0.23 kg. of gelatin was kneaded. This mixture was added to a solution of heptane containing 0.28 kg. of polyisobutylene (Vistanex LM-MH), 0.31 kg. of piccolyte resin, 8 g. of butylated hydroxytoluene, and 0.13 kg. of mineral oil to form an adhesive slurry. A portion of this slurry was poured onto a sheet of silicone coated release paper and the solvent evaporated to leave an adhesive layer of about 0.015 inches (0.38 mm) in thickness. This adhesive layer was laminated to a semi-open cell polyurethane foam of 0.065 inches (1.65 mm) thickness having a polyurethane skin of about 0.002 inches (0.05 mm) thickness on one surface by gently compressing the adhesive layer to the foam by passing through pressure rollers.

The silicone coated release paper was then stripped from adhesive layer 13 and adhesive layer 13 and adhesive layer 14 were compressed together by passing through pressure rollers. Silicone coated release paper was then pressed onto the exposed surface of adhesive layer 14. The resulting dressing was cut into the desired shape and packaged.

Examples 2 - 15

Following the procedure of Example 1 but employing the following ingredients on a weight percent basis in adhesive layer 14 other dressings within the scope of this invention are obtained.

| Ingredient | Example | | | |
|---|---|---|---|---|
| | 2 | 3 | 4 | 5 |
| Polyisobutylene (Vistanex LM-MH) | 40 | 40 | 40 | 40 |
| Guar gum | 25 | 60 | - | 25 |
| Locust bean gum | - | - | - | - |
| Pectin | - | - | - | - |
| Karaya | - | - | - | - |
| Gelatin | - | - | - | - |
| Sodium carboxymethylcellulose | 10 | - | 17.2 | 10 |
| Collagen | - | - | - | - |
| Cross-linked sodium carboxymethylcellulose (Aqualon R) | 15 | - | 25.6 | 15 |
| Starch-acrylonitrile graft copolymer (Grain Processing Corp. Polymer 35-A-100) | - | - | - | - |
| Cross-linked dextran (Sephadex CM-C50) | - | - | - | - |
| Poly(methyl vinyl ether/maleic acid), mixed calcium, sodium salt (Gantrez AT-955) | 10 | - | 17.2 | 10 |
| Polyisobutylene (Vistanex L-100) | - | - | - | - |
| Butyl rubber (grade 077) | - | - | - | - |
| Styrene-isoprene copolymer (Kraton 1107) | - | - | - | - |

| Ingredient | Example | | | | |
|---|---|---|---|---|---|
| | 6 | 7 | 8 | 9 | 10 |
| Polyisobutylene (Vistanex LM-MH) | 40 | 40 | 45 | 50 | 20 |
| Guar gum | 30 | 30 | 25 | - | 20 |
| Locust bean gum | - | - | - | 20 | - |
| Pectin | - | - | 15 | - | - |
| Karaya | - | - | - | 20 | - |
| Gelatin | - | - | - | - | - |
| Sodium carboxymethylcellulose | 12 | - | - | - | 10 |
| Collagen | - | 10 | - | - | 10 |
| Cross-linked sodium carboxymethylcellulose (Aqualon R) | 18 | - | - | - | 15 |
| Starch-acrylonitrile graft copolymer (Grain Processing Corp. Polymer 35-A-100) | - | - | 15 | - | - |
| Cross-linked dextran (Sephadex CM-C50) | - | - | - | 10 | - |
| Poly(methyl vinyl ether/maleic acid), mixed calcium, sodium salt (Gantrez AT-955) | - | 20 | - | - | - |
| Polyisobutylene (Vistanex L-100) | - | - | - | - | 25 |
| Butyl rubber (grade 077) | - | - | - | - | - |
| Styrene-isoprene copolymer (Kraton 1107) | - | - | - | - | - |

| Ingredient | Example | | | | |
|---|---|---|---|---|---|
| | 11 | 12 | 13 | 14 | 15 |
| Polyisobutylene (Vistanex LM-MH) | 30 | 20 | 40 | 40 | 40 |
| Guar gum | - | 20 | - | 20 | - |
| Locust bean gum | - | - | - | - | 20 |
| Pectin | 15 | - | 15 | - | 20 |
| Karaya | - | - | - | - | - |
| Gelatin | 15 | - | 15 | - | - |
| Sodium carboxymethylcellulose | 15 | 20 | 15 | 15 | 10 |
| Collagen | - | - | 15 | 15 | - |
| Cross-linked sodium carboxymethylcellulose (Aqualon R) | 10 | - | - | 10 | - |
| Starch-acrylonitrile graft copolymer (Grain Processing Corp. Polymer 35-A-100) | - | - | - | - | 10 |
| Cross-linked dextran (Sephadex CM-C50) | - | 20 | - | - | - |
| Poly(methyl vinyl ether/maleic acid), mixed calcium, sodium salt (Gantrez AT-955) | - | - | - | - | - |
| Polyisobutylene (Vistanex L-100) | - | - | - | - | - |
| Butyl rubber (grade 077) | 15 | - | - | - | - |
| Styrene-isoprene copolymer (Kraton 1107) | - | 20 | - | - | - |

Examples 16 - 23

Following the procedure of Example 1 but employing the ingredients listed below on a weight percent basis in adhesive layer 13 other dressings within the scope of this invention are obtained.

| Ingredient | Example | | | |
|---|---|---|---|---|
| | 16 | 17 | 18 | 19 |
| Polyisobutylene (Vistanex LM-MH) | 15 | 15 | 15 | 15 |
| Guar gum | 20 | 15 | - | - |
| Locust bean gum | - | - | 17 | - |
| Pectin | - | - | - | 10 |
| Karaya | - | - | - | - |
| Gelatin | - | - | - | 10 |
| Sodium carboxymethylcellulose | 15 | 15 | 10 | 10 |
| Collagen | - | - | - | - |
| Cross-linked sodium carboxymethylcellulose (Aqualon R) | - | 15 | - | - |
| Starch-acrylonitrile graft copolymer (Grain Processing Corp. Polymer 35-A-100) | - | - | - | - |
| Cross-linked dextran (Sephadex CM-C50) | - | - | - | 10 |
| Poly(methyl vinyl ether/maleic acid), mixed calcium, sodium salt (Gantrez AT-955) | - | - | 10 | - |
| Polyisobutylene (Vistanex L-100) | - | - | 20 | 15 |
| Butyl rubber (grade 077) | 20 | - | - | - |
| Styrene-isoprene copolymer (Kraton 1107) | - | 19.5 | - | - |
| Mineral oil | 9.5 | 5 | 7.5 | 9.5 |
| Picolyte resin | 20 | 15 | 20 | 20 |
| Butylated hydroxytoluene | 0.5 | 0.5 | 0.5 | 0.5 |

| Ingredient | Example | | | |
|---|---|---|---|---|
| | 20 | 21 | 22 | 23 |
| Polyisobutylene (Vistanex LM-MH) | 15 | 15 | 20 | 15 |
| Guar gum | 15 | 15 | 25 | - |
| Locust bean gum | - | - | - | - |
| Pectin | - | - | - | - |
| Karaya | - | - | - | 10 |
| Gelatin | - | - | - | - |
| Sodium carboxymethylcellulose | 10 | - | - | 15 |
| Collagen | - | - | 5 | - |
| Cross-linked sodium carboxymethylcellulose (Aqualon R) | - | 10 | - | - |
| Starch-acrylonitrile graft copolymer (Grain Processing Corp. Polymer 35-A-100) | 10 | - | - | - |
| Cross-linked dextran (Sephadex CM-C50) | - | - | - | 10 |
| Poly(methyl vinyl ether/maleic acid), mixed calcium, sodium salt (Gantrez AT-955) | - | 10 | - | - |
| Polyisobutylene (Vistanex L-100) | 20 | 20 | 15 | 20 |
| Butyl rubber (grade 077) | - | - | - | - |
| Styrene-isoprene copolymer (Kraton 1107) | - | - | - | - |
| Mineral oil | 9.5 | 9.5 | 9.5 | 9.5 |
| Picolyte resin | 20 | 20 | 25 | 20 |
| Butylated hydroxytoluene | 0.5 | 0.5 | 0.5 | 0.5 |

Small amounts, i.e., less than 5% by weight, of other ingredients may be included within or sprayed onto the granules. For example, an antioxidant such as butylated hydroxyanisole or butylated hydroxytoluene, a deodorant such as chlorophyllins, or a perfume agent may be included. In addition, small amounts of a pharmacologically active ingredient can be included within or sprayed onto the granules. For example, an antibiotic or antimicrobial agent such as neomycin, an antiseptic agent such as povidone iodine, and an antiinflammatory agent such as hydrocortisone or triamcinolone acetonide.

The granules preferably contain at least 70% by weight of one or more water dispersable hydrocolloids selected from pectin, gelatin, sodium carboxymethylcellulose, and collagen with a granular product consisting of an equal weight percent mixture of pectin, gelatin, and sodium carboxymethylcellulose being most preferred.

The granular material can be prepared from the powder ingredients by dry compaction, wet granulation, or fluidized bed granulation techniques. The dry compaction method involves blending the component powders, compacting into a slab, and milling the slab to the desired particle size. The milled material is sieved and particles of the proper size range are gathered and packaged.

In the wet granulation process, the powders after blending, or simultaneously with blending, are moistened with water, a hydroalcoholic solution, or low concentration dispersion of one or more of the water dispersable hydrocolloids in water or hydroalcoholic vehicles. Normally, the amount of moisture added is up to about 50% of the dry weight of the powders in order to form granules with adequate process-ability and resistance to attrition. After thorough mixing, the moistened mass is forced through a screen or die to yield granules directly or to yield by extrusion, a particulate noodle-like or ribbon-like mass of material. When dried, this material is then milled to the desired sieve size and packaged. Alternatively, the moistened mass may first be broken into large lumps which are then dried and milled to the desired particle size.

In the fluid bed granulation procedure, the moistening fluid is added to a charge of the blended powders held suspended on a column of rising warm air where the powders are allowed to mix with the granulating fluid, which is very rapidly evaporated, leaving behind agglomerated granules. Less moistening fluid is required in this process then in the wet granulation process described above.

Example 24

To 120 kg. of a mixture of equal parts of gelatin, pectin and sodium carboxymethylcellulose were added about 50 liters of distilled water or a mixture of water and ethanol. Mixing was performed in a rotating oscillating device to produce a homogeneous blend. The blend was screened through a coarse sieve (0.5 inch = 12.7 mm) and the moistened material was dried at 55°C for 24 hours. The dried material was milled and screened to between 16 and 20 mesh (1.19 and 0.84 mm openings).

Examples 25 - 30

Following the procedure of Example 24 but employing the ingredients listed below on a weight percent basis other granular products within the scope of this invention are obtained.

| Ingredient | Example | | | |
|---|---|---|---|---|
| | 25 | 26 | 27 | 28 |
| Guar gum | - | - | - | - |
| Locust bean gum | - | - | - | - |
| Pectin | 25 | 50 | - | - |
| Karaya | - | - | - | - |
| Gelatin | 25 | - | 50 | - |
| Sodium carboxymethylcellulose | 25 | - | - | 50 |
| Calcium carboxymethylcellulose | - | - | - | - |
| Collagen | 25 | 50 | 50 | 50 |
| Cross-linked sodium carboxymethylcellulose (Aqualon R) | - | - | - | - |
| Starch-acrylonitrile graft copolymer (Grain Processing Corp. Polymer 35-A-100) | - | - | - | - |
| Cross-linked dextran (Sephadex CM-C50) | - | - | - | - |
| Poly(methyl vinyl ether/maleic acid), mixed calcium, sodium salt (Gantrez AT-955) | - | - | - | - |

| Ingredient | Example | |
|---|---|---|
| | 29 | 30 |
| Guar gum | 20 | - |
| Locust bean gum | - | 20 |
| Pectin | 20 | - |
| Karaya | - | 20 |
| Gelatin | - | - |
| Sodium carboxymethylcellulose | 40 | 60 |
| Calcium carboxymethylcellulose | 20 | - |
| Collagen | - | - |
| Cross-linked sodium carboxymethylcellulose (Aqualon R) | - | - |
| Starch-acrylonitrile graft copolymer (Grain Processing Corp. Polymer 35-A-100) | - | - |
| Cross-linked dextran (Sephadex CM-C50) | - | - |
| Poly(methyl vinyl ether/maleic acid), mixed calcium, sodium salt (Gantrez AT-955) | - | - |

The use of the occlusive dressings described above results in a closed moist wound treatment environment. Unlike gauze type dressings, the ingredients employed in adhesive layer 14 permit the dressings of this invention to remain in place over the wound for up to several days. It is believed that the need to frequently change a dressing disturbs the wound healing environment and results in a slower healing process.

The water dispersable hydrocolloid materials, the water swellable cohesive strengthening agents, and the hydratable polymers distributed throughout adhesive layer 14 react in the presence of moisture. In the area of normal skin surrounding the wound site, the layer 14 will gradually hydrate over a period of days. The initial aggressive bond of the dressing to this normal skin is due to the presence of the pressure sensitive adhesive materials in layer 14; i.e., dry tack. As this bond is lessened by perspiration and leakage of moisture under layer 14, the wet tack of the moisture active ingredients in layer 14 becomes more critical in bonding the dressing to the skin. Eventually, layer 14 becomes so hydrated that the dressing can be removed without stripping or macerating the skin around the wound site.

When the dressing is applied over a fluid emitting wound such as a dermal ulcer, it has been observed that the moisture active ingredients in layer 14 will be converted to an almost gel-like mass. This provides an ideal moist environment for cell migration ensuring easy dressing removal with a minimum of damage to the newly formed tissues.

In treating ulcers emitting a large volume of exudate, it has been found to be useful to first pack the wound with the granular material described above and then cover with the occlusive dressing. The granules interact with the wound exudate to form a gel-like mass and prevent leakage through the dressing. As a result, the dressing is changed less often which is believed to result in a shortened healing period. After removal of the dressing, the hydrated granules can be removed from the wound site by flushing with saline solution. As healing progresses and less exudate is present in the sound site, the dressing can be employed without the granular packing.

Fig. 7 shows an ulcer 70 covered by occlusive dressing 10. The dressing is partially broken away to show the granular packing material 75. Of course,adhesive layer 14 contacts the granules 75 in the area of the wound and also bonds the dressing to the normal skin 71 surrounding the ulcer.

## Claims

1. A granular product for treating wound exudate, having a particle size of from about 10 to about 40 mesh (from 2.00 to 0.42 mm sieve openings) consisting essentially of one or more water dispersible hydrocolloids selected from pectin, gelatin, sodium carboxymethylcellulose, collagen, guar gum, locust bean gum, karaya, and calcium carboxymethylcellulose, wherein the granular product is prepared from the above powder ingredients by a dry compaction, wet granulation, or fluidized bed granulation technique.

2. The product of claim 1 consisting of an equal weight percent mixture of pectin, gelatin, and sodium carboxymethylcellulose.

3. A method of preparing a granular product for treating wound exudate, having a particle size of from about 10 to about 40 mesh (from 2.00 to 0.42 mm sieve openings) consisting essentially of one or more water dispersible hydrocolloids selected from pectin, gelatin, sodium carboxymethyl-cellulose, collagen, guar gum, locust bean gum, karaya, and calcium carboxymethylcellulose, wherein the granular product is prepared from the above powder ingredients by a dry compaction, wet granulation, or fluidized bed granulation technique.

4. A method according to claim 3 wherein the product consists of an equal weight percent mixture of pectin, gelatin, and sodium carboxymethylcellulose.

## Patentansprüche

1. Granulat zur Wundexudatbehandlung mit einer Teilchengröße von etwa 10 bis etwa 40 mesh (2,00 bis 0,42 mm Sieböffnungen), im wesentlichen bestehend aus einem oder mehreren in Wasser dispergierbaren Hydrokolloiden, ausgewählt aus Pectin, Gelatine, Natriumcarboxymethylcellulose, Kollagen, Guargummi, Johannesbrotgummi, Karaya und Calciumcarboxymethylcellulose, wobei das Granulat aus den vorstehend genannten pulverförmigen Inhaltsstoffen durch Trockenverpressen, Naßgranulierung oder Wirbelschichtgranulierung hergestellt wird.

2. Produkt nach Anspruch 1, bestehend aus einem Gemisch von Pectin, Gelatine und Natriumcarboxymethylcellulose gleichen prozentualen Gewichts.

3. Verfahren zur Herstellung eines Granulats zur Wundexudatbehandlung mit einer Teilchengröße von etwa 10 bis etwa 40 mesh (2,00 bis 0,42 mm Sieböffnungen), im wesentlichen bestehend aus einem oder mehreren in Wasser dispergierbaren Hydrokolloiden, ausgewählt aus Pectin, Gelatine, Natriumcarboxymethylcellulose, Kollagen, Guargummi, Johannisbrotgummi, Karaya und Calciumcarboxymethylcellulose, wobei das Granulat aus den vorstehend genannten pulverförmigen Inhaltsstoffen durch Trockenverpressen, Naßgranulierungn oder Wirbelschichtgranulierung hergestellt wird.

4. Verfahren nach Anspruch 3, wobei das Produkt aus einem Gemisch von Pectin, Gelatine und Natriumcarboxymethylcellulose gleichen prozentualen Gewichts besteht.

## Revendications

1. Produit granuleux de traitement d'exsudat de plaie, ayant une granulométrie d'environ 10 à environ 40 mesh (tamis à ouverture de maille de 2,00 à 0,42 mm), se composant essentiellement d'un ou plusieurs hydrocolloïdes dispersibles dans l'eau choisis entre la pectine, la gélatine, la carboxyméthylcellulose sodique, le collagène, le guar, la gomme tragasol, le karaya et la carboxyméthylcellulose calcique, dans lequel le produit granuleux est préparé à partir des ingrédients en poudre ci-dessus par une technique de compactage à sec, de granulation à l'état humide ou de granulation en lit fluidisé.

2.    Produit selon la revendication 1, se composant d'un mélange, en pourcentages pondéraux égaux, de pectine, de gélatine et de carboxyméthylcellulose sodique.

3.    Procédé de préparation d'un produit granuleux de traitement d'exsudat de plaie, ayant une granulométrie d'environ 10 à environ 40 mesh (tamis à ouverture de maille de 2,00 à 0,42 mm), se composant essentiellement d'un ou plusieurs hydrocolloïdes dispersibles dans l'eau choisis entre la pectine, la gélatine, la carboxyméthylcellulose sodique, le collagène, le guar, la gomme tragasol, le karaya et la carboxyméthylcellulose calcique, dans lequel le produit granuleux est préparé à partir des ingrédients en poudre ci-dessus par une technique de compactage à sec, de granulation à l'état humide ou de granulation en lit fluidisé.

4.    Procédé selon la revendication 3, dans lequel le produit se compose d'un mélange, en pourcentages pondéraux égaux, de pectine, de gélatine et de carboxyméthylcellulose sodique.

*Fig. 1*

*Fig. 2*

*Fig. 3*

Fig. 4

Fig. 5

Fig. 6

Fig. 7